(19) Europäisches Patentamt
European Patent Office
Office européen des brevets

(11) EP 1 795 607 A1

(12) EUROPEAN PATENT APPLICATION

(43) Date of publication:
**13.06.2007 Bulletin 2007/24**

(51) Int Cl.:
***C12Q 1/46*** *(2006.01)*

(21) Application number: **05026822.6**

(22) Date of filing: **08.12.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
- **Roche Diagnostics GmbH**
  **68305 Mannheim (DE)**
  Designated Contracting States:
  **DE**
- **F.HOFFMANN-LA ROCHE AG**
  **4070 Basel (CH)**
  Designated Contracting States:
  **AT BE BG CH CY CZ DK EE ES FI FR GB GR HU IE IS IT LI LT LU LV MC NL PL PT RO SE SI SK TR**

(72) Inventors:
- **Weber, Friederike**
  **80803 München (DE)**
- **Nagel, Rolf**
  **68642 Buerstadt (DE)**
- **Kytzia, Hans-Joachim Dr.**
  **79227 Schallstadt (DE)**
- **Muecke, Matthias Dr.**
  **82377 Penzberg (DE)**
- **Gottschalk, Norbert Dr.**
  **69115 Heidelberg (DE)**

Remarks:
Amended claims in accordance with Rule 86 (2) EPC.

# (54) Stabilized cholinesterase substrate solution

(57) The present invention provides a stabilized cholinesterase substrate solution, wherein at least one substrate is stabilized by at least one species of a polar organic solvent. The present invention also relates to the use of a stabilized cholinesterase substrate solution, wherein at least one substrate is stabilized by at least one species of a polar organic solvent. Also within the scope of the present invention is a stabilized cholinesterase substrate solution for determining the activity of a cholinesterase in a sample, wherein at least one substrate is stabilized by at least one species of a polar organic solvent.

Furthermore, the invention relates to a method for determining the activity of a cholinesterase in a sample, as well as a kit for determining the activity of a cholinesterase in a sample and a kit for conducting a method for determining the activity of a cholinesterase in a sample.

EP 1 795 607 A1

## Description

### Field of the invention

The present invention relates to the field of measuring enzymatic activities in biological samples. More specifically, it provides a stabilized solution of a cholinesterase substrate and the use of said solution for determining the activity of cholinesterase in a sample. Furthermore, methods and kits for determining the activity of cholinesterase are provided.

### Background of the invention

Cholinesterase is the common name for an enzymatic activity, which catalyzes the hydrolysis of an acylester to an alcohol and a carboxylate. Cholinesterases act on a variety of substrates with alcohol moieties such as phenyl-, indoxyl-alcohols, and preferably choline and with carboxylate moieties such as carbonic, dicarbonic and benzoic acids (Brown et al., Adv. Clin. Chem., 22:1-123 (1981)). Based on their specificity towards carboxylate moieties, cholinesterases are subdivided into two groups. The "true cholinesterases" or acetylcholinesterases (acetylcholine acetylhydrolase, EC 3.1.1.7) show a marked selectivity for acetyl moieties, whereas the "pseudocholinesterases" or acylcholinesterases (acylcholine acylhydrolase, EC 3.1.1.8) preferably hydrolyse substrates with bigger carboxylate moieties such as butyryl or benzoyl.

Acetylcholinesterase plays a most important role in signal transduction within the nervous system and between nerve and muscle. It is also present in erythrocytes. The enzyme is irreversably inactivated by nerve gases such as sarin, but also in insecticide poisoning.

The biological function of acylcholiziesterase is unknown. Acylcholinesterase can be found in pancreas, heart, intestinal mucosa, serum, in the white brain substance and in liver.

Cholinesterases and the determination of their activity particularly has become of clinical interest. Due to its obvious hepatic origin, this mainly applies to acylcholinesterase. Detected as an index of liver function, the enzyme is also used as a clinical indicator for suspected insecticide toxication. By a preoperative screening for acylcholinesterase, patients with atypical forms of the enzyme can be identified aiming at preventing a prolonged apnea, which is caused by a delayed degradation of muscle relaxans. Decreased levels of acylcholinesterase can be found in connection with toxications by phosphoorganic compounds, hepatitis, cirrhosis, myocard infarct, acute infections and with atypic phenotypes of the enzyme.

Methods for the detection of cholinesterase activity in biological samples are described in the prior art (Burtis and Ashwood (eds.), Tietz fundamentals of clinical chemistry -4th ed., ISBN 0-7216-3763-9). The common test principle is based on the use of thiocholine esters such as acetylthiocholine, propionylthiocholine, butyrylthiocholine and succinylbisthiocholine. Among those, only acetyl- and propionylthiocholine are useful in the determination of acetylcholinesterase. Serum acylcholinesterase, although active against all these substrates, is preferably determined using succinylbisthiocholine and especially butyrylthiocholine. All these thioesters are unstable in aqueous solution because of hydrolysis of the thioester bond. The low stabilities of refrigerated reagent solutions have been well established by several authors, e.g. acetylthiocholine, 7 days (Whittaker M. Cholinesterases. In: Bergmeyer HU, ed; Methods of Enzymatic Analyses, 3rd edition, Weinheim Verlag Chemie, 1984:52-74); propionylthiocholine, 1 day (Dietz et al. Colorimetric Determination of Serum Cholinesterase and Its Genetic Variance by the Propionylthiocholine-Dithiobis(nitrobenzvic Acid) Procedure. Clinical Chemistry 1973;19:1309-1313); butyrylthiocholine, 30 days (Schmidt E. et al. Proposal of Standard Methods for the Determination of Enzyme Catalytic Concentrations in Serum and Plasma at 37°C. Eur.J.Clin.Chem.Clin.Biochem.1992;30.163-170). Whilst some of the aforementioned substrates of acylcholinesterase are also split by acetylcholinesterase, arylesterase (arylester hydrolase, EC 3.1.1.2), and even albumin, butyrylthiocholine (BTC; Knedel and Böttger, Klin Wschr 45:325-327 (1967)) does not suffer from those drawbacks. Thus BTC is generally regarded as one of the most specific substrates and was chosen for national recommended cholinesterase assay methods (Association of Clinical Biochemists, News Sheet Assoc. Clin. Biochemists, Suppl. 202:31s-36s (1980); Cholinesterase, In: Kommentare zum Arzneibuch der Deutschen Demokratischen Republik, Heft 2: Enzym-Aktivitätsbestimmung in der Laboratoriumsdiagnostik, Akademie-Verlag, Berlin, 56-65 (1988)). Dependent on acylcholinesterase activity, BTC is hydrolyzed to thiocholine and butyrate. Thiocholine reacts with 5,5'-dithiobis-2-nitrobenzvate (DTNB) leading to the formation of the yellow dye 5-mercapto-2-nitrobenzoate.

S-butyrylthiocholine + $H_2O$ —Cholinesterase→ butyric acid + thiocholine

thiocholine + DTNB → 5-mercapto-2-nitrobenzoate + 2-nitrobenzoate-mercapto-thiocholine

The formation rate of 5-mercapto-2-nitrobenzoate is directly proportional to the catalytic activity of the acylcholinesterase, which is determined according to the increase in the extinction at a wavelength of 480nm. Due to the specificity of the substrate, the test performed with serum is not interfered by acetylcholinesterase, which is released from erythrocytes because of marginal hemolysis.

In another acylcholinesterase assay, the test principle by Knedel and Böttger, Klin. Wschr. 45:325-327 (1967) is modified in that thiocholine as product of the acylcholinesterase-driven reaction instantaneously reduces yellow hexacyanoferrate (III) to almost colourless hexacyanoferrate (II), thus also allowing the direct spectrometric monitoring of the reaction.

Optimized reaction conditions for measuring the catalytic activity of acylcholinesterase in human sera have been investigated and established (Schmidt et al., Eur. J. din. Chem. Clin. Biochem. 30(3): 163-170 (1992)). These conditions consider both the enzyme kinetics and the technical aspects of manual and mechanized performance. They do not necessarily provide maximum possible conversion rates, but only the highest available robustness of the method.

However, an essential and yet unsolved problem is the low stability of BTC, since butyrylthiocholine as a thioester compound is prone autohydrolysis. Consequently the spontaneous cleavage of BTC under assay conditions always has to be subtracted from the turnover of BTC to get the real acylcholinesterase activity. It is known that this reagent blank reaction can be minimized to a certain extent by optimizing substrate concentration and buffer conditions. Since the stability of BTC in solution significantly decreases with increasing storage time, an essential prerequisite thereby is the provision of a freshly prepared BTC solution. At present this can only be achieved by the reconstitution of a BTC granulate, a process which is intricate and time-consuming.

There is no reagent available in the prior art, which allows the storage of dissolved cholinesterase subtrates such as BTC over a longer time period in absence of any enzyme without having an increasing autohydrolysis activity. Due to the need to provide a freshly dissolved substrate in order to minimize the reagent blank reaction in a cholinesterase assay, a detection of cholinesterase activities in a larger set of samples in parallel would be complex and ineffective. A simple and effective detection of cholinesterase activities in numerous samples, e.g. by performing a high throughput process in an automated analyzer, is not possible at the time.

Hence, the technical problem underlying the present invention is to provide a stabilized cholinesterase substrate solution.

**Summary of the invention**

The object of the present invention is a stabilized cholinesterase substrate solution, wherein at least one substrate is stabilized by at least one species of a polar organic solvent

The present invention also relates to the use of a stabilized cholinesterase substrate solution, wherein at least one substrate is stabilized by at least one species of a polar organic solvent. Also within the scope of the present invention is a stabilized cholinesterase substrate solution for determining the activity of a cholinesterase in a sample, wherein at least one substrate is stabilized by at least one species of a polar organic solvent.

Furthermore, the invention relates to a method for determining the activity of a cholinesterase in a sample, comprising the steps of combining a sample suspected of containing the enzyme under conditions suitable for enzymatic activity and a reagent comprising a stabilized cholinesterase substrate solution, wherein at least one substrate is stabilized by at least one species of a polar organic solvent, and of monitoring the activity of the enzyme,

Within the scope of the present is a kit for determining the activity of a cholinesterase in a sample comprising in a packaged combination a first reagent comprising a buffer solution suitable for the activity of cholinesterase, and a second reagent comprising a stabilized cholinesterase substrate solution, wherein at least one substrate is stabilized by at least one species of a polar organic solvent. Also within the scope of the present invention is a kit for conducting a method for determining the activity of a cholinesterase in a sample, the method comprising the steps of combining a sample suspected of containing the enzyme under conditions suitable for enzymatic activity and a reagent comprising a stabilized cholinesterase substrate solution, wherein at least one substrate is stabilized by at least one species of a polar organic solvent, and of monitoring the activity of the enzyme.

## Brief description of the drawings

Fig.1 shows the comparison of the catalytical concentration of cholinesterase (CHE) obtained with freshly prepared CHE substrate solution stabilized with DMSO (20% vol.) and with the same stabilized solution tempered at 35°C for 18 days.

Fig. 2 shows the comparison of the catalytical concentration of cholinesterase (CHE) obtained with freshly prepared CHE substrate solution according to the proposal of the DGKC (Deutsche Gesellschaft fur Klinische Chemie, see Schmidt et al., Eur. J. Clin. Chem. Clin. Biochem. 30(3): 163-170 (1992) and with the same solution tempered at 35°C for 21 days.

## Detailed description of the invention

Surprisingly, it has been found that the combination of at least one cholinesterase substrate with at least one species of a polar organic solvent results in a cholinesterase substrate solution, which is stabilized. Accordingly, the present invention provides a stabilized cholinesterase substrate solution, wherein at least one substrate is stabilized by at least one species of a polar organic solvent.

The term "stabilized" means that the cholinesterase substrate solution according to the present invention can be stored in absence of any enzyme activity without a significant inactivation of the substrate.

"Without a significant inactivation of the substrate" means that e.g. after a storage of the stabilized solution at 35°C over a time period of at least 18 days or after a storage under typical laboratory conditions, that is refrigerated at 4-8°C for a time period of at least 15 months no loss of BTC has occurred, resulting in an intercept of about <+/-300 U/l and a slope <+/- 3% of a straight-line in a plot of CHE catalytic concentrations obtained using freshly prepared and incubated substrate solution.

The term "cholinesterase" comprises an enzymatic activity, which catalyzes the hydrolysis of an acylester to an alcohol and a carboxylate. In the scope of the present invention any cholinesterase is comprised, preferably acetylcholinesterase as defined by EC 3.1.1.7 or acylcholinesterase as defined by EC 3.1.1.8. It is also to be understood that "cholinesterase" is meant to cover cholinesterase as well as its biologically active subforms, fragments and subunits, which behave in a biological sense, i.e. via hydrolyzation of an acylester to an alcohol and a carboxylate, as cholinesterase.

A stabilized cholinesterase substrate solution according to the invention has striking advantages in comparison with the hitherto known cholinesterase substrate solutions which have to be reconstituted from granulates prior to application. Being a liquid reagent, for a stabilized substrate solution according to the invention there is not any longer a need for the reconstitution from a granulate. This is an essential prerequisite for a constant accuracy of the detection of cholinesterase activity. A reconstitution, i.e. bringing granulate in solution is usually time consuming and error-prone. After each reconstitution, an additional calibration assay has to be performed to verify experimental data obtained with independently reconstituted substrate solutions. In contrast, the provision of a stabilized cholinesterase substrate solution allows the homogeneity of the composition over all bottles of a reagent lot thereby guaranteeing an improvement of accuracy and a reduction of the number of calibration assays.

In an embodiment of the present invention the stabilized cholinesterase substrate solution is a stabilized acylcholinesterase substrate solution. I another embodiment of the present invention the stabilized cholinesterase substrate solution is a stabilized acetylcholinesterase substrate solution.

The term "cholinesterase substrate" comprises all substrates, which are hydrolyzed by cholinesterase, comprising substrates belonging to the class of acyl esters. In an embodiment of the present invention the at least one substrate of a stabilized cholinesterase substrate solution is a choline ester. In another embodiment the at least one substrate of the stabilized cholinesterase substrate solution is a thioester, preferably butyrylthiocholine (BTC) or acetylthiocholine (ATC).

The term "polar organic solvent" refers to solvents consisting of organic compounds, which contain carbon atoms and which have hydrophilic properties due to unequally distributed electric charges in the organic compounds, leaving one end of each molecule more positive than the other. In an embodiment of the present invention said at least one species of a polar organic solvent is a polar protic organic solvent. The term "polar protic organic solvent" refers to organic solvents, the organic compounds of which contain, in contrast to a "polar aprotic organic solvent", one or more acidic hydrogen atoms. In a preferred embodiment of the invention, said at least one species of a polar protic organic solvent is an alcohol.

The term "alcohol" refers to univalent alcohols, i.e. alcohols with only one hydroxyl group, as well as to polyvalent alcohols containing more than one hydroxyl group. Preferably the at least one species of a polar protic organic solvent is an alcohol selected from the group with a chain length between 1 and 7 carbon atoms. In the scope of the present

invention, alcohols with a chain length between 1 and 7 carbon atoms comprise alcohols with a branched carbon chain such as 3-Methylhexan-3-ol as well as alcohols with a linear carbon chain such as ethanol. In a preferred embodiment of the present invention, the stabilized cholinesterase substrate solution is characterized in that at least one substrate is stabilized by an alcohol selected from the group consisting of methanol, ethanol, propanol and isopropanol. In another preferred embodiment of the invention, the stabilized cholinesterase substrate solution is stabilized by glycerine.

According to the present invention, said at least one species of a polar organic solvent may also comprise a carboxylic acid, The term "carboxylic acid" refers to organic acids characterized by the presence of a carboxyl group. "Carboxylic acid" also comprises the salts and anions of carboxylic acids, which are called carboxylates, as well as carboxylic acids having two or more carboxylic acid groups per molecule.

In an embodiment of the present invention said at least one species of a polar organic solvent is a polar aprotic organic solvent, preferably selected from the group consisting of carbonyl compounds or heterocarbonyl compounds. In a more preferred embodiment of the invention, said carbonyl compound or heterocarbonyl compound is selected from the group containing 1 to 12 carbon atoms. In another preferred embodiment of the present invention, the stabilized cholinesterase substrate solution is characterized in that at least one substrate is stabilized by a carbonyl compound or a heterocarbonyl compound selected from the group consisting of dimethylsulfoxide (DMSO), dimethylformamid (DMF), acetone, acetylacetone, acetonitril and hexamethylphosphoric triamide (HMPT).

In another preferred embodiment of the present invention, said at least one species of a polar aprotic organic solvent is an ether. The term "ether" refers to a class of chemical compounds which contain an ether group i.e. an oxygen atom connected to two substituted alkyl groups. In a more preferred embodiment of the present invention, the stabilized cholinesterase substrate solution is characterized in that at least one substrate is stabilized by an ether selected from the group containing 1 to 12 carbon atoms. In the scope of the present invention, any ether containing 1 to 12 carbon atoms is comprised, e.g. cyclic ether as well as ether having branched or linear carbon chains. In an embodiment of the present invention, the stabilized cholinesterase substrate solution is characterized in that at least one substrate is stabilized by a cyclic ether, preferably tetrahydrofuran (THF) or dioxan.

Also within the scope of the present invention is a stabilized cholinesterase substrate solution, wherein the concentration of at least one species of a polar organic solvent in that reaction mixture is at least 0.25 volume percent and maximal 100 volume percent.

In a preferred embodiment of the present invention, the concentration of the at least one species of a polar organic solvent in a stabilized cholinesterase substrate solution is for DMSO at least 0.25 volume percent and maximal 100 volume percent, preferably at least 0.25 volume percent and maximal 20 volume percent, for ethanol at least 0.25 volume percent and maximal 100 volume percent, preferably at least 0.25 volume percent and maximal 20 volume percent, for isopropanol at least 0.5 volume percent and maximal 95 volume percent, preferably at least 0.5 volume percent and maximal 20 volume percent, and/or for THF at least 0.5 volume percent and maximal 100 volume percent, preferably at least 0.5 volume percent and maximal 20 volume percent.

Another aspect of the present invention is the use of a stabilized cholinesterase substrate solution, wherein at least one substrate is stabilized by at least one species of a polar organic solvent. Also within the scope of the present invention is a stabilized cholinesterase substrate solution for determining the activity of a cholinesterase in a sample, wherein at least one substrate is stabilized by at least one species of a polar organic solvent.

The term "sample" refers to any sample suspected of containing cholinesterase. The sample is typically an aqueous solution. In an embodiment of the present invention, the sample is a biological sample, such as a body fluid from a host, for example, urine, whole blood, plasma, serum, saliva, semen, stool, sputum, cerebral spinal fluid, tears, mucus or the like, but preferably is plasma or serum. The sample can be pretreated if desired and can be prepared in any convenient medium that does not interfere with the assay. An aqueous medium is preferred.

The activity of cholinesterase can be determined by quantitative, semi-quantitative and qualitative methods, as well as by all other methods for determining cholinesterase. For example, a method that merely detects the presence or absence of cholinesterase in a sample suspected of containing cholinesterase is considered to be included within the scope of the present invention. The terms "detecting", "monitoring" and "measuring", as well as other common synonyms for determining, are contemplated within the scope of the present invention.

The determination of cholinesterase activity according to the present invention may be conducted by a rate-assay method wherein change in absorbance of a product of the cholinesterase activity per unit time is measured or by an end-point method wherein the reaction is quenched after a certain period of time has elapsed. According to the present invention the determination of cholinesterase activity also may be conducted by measuring change in absorbance of a product, which results from a secondary reaction starting from one of the products of the actual enzymatic reaction. The method can easily be applied to automated analyzers for laboratory or clinical analysis. In an embodiment of the present invention the monitoring of the activity of cholinesterase is conducted by determining the formation of thiocholine, preferably by measuring the reduction of hexacyanoferrate (III) to hexacyanoferrate (II) by spectrophotometry. In another preferred embodiment of the present invention, the cholinesterase-dependent formation of thiocholine is determined by measuring the formation of 5-mercapto-2-nitrobenzoate as a product of the reaction of thiocholine with 5,5’-dithiobis-2-nitrobenzoate by spectrophotometry. According to the present invention, the monitoring of the activity of cholinesterase may be conducted by using an automated analyzer.

Also within the scope of the present invention is a method for determining the activity of a cholinesterase in a sample, comprising the steps of combining a sample suspected of containing the enzyme under conditions suitable for enzymatic activity and a reagent comprising a stabilized cholinesterase substrate solution, wherein at least one substrate is stabilized by at least one species of a polar organic solvent, and of monitoring the activity of the enzyme.

To enhance the versatility of the invention, reagents useful in the methods of the invention can be provided in a packaged combination, in the same or in separate containers, in liquid or in lyophilized form so that the ratio of the reagents provides for substantial optimization of the method and assay. The reagents may each be in separate containers or various reagents can be combined in one or more containers depending on the eross-reactivity and the stability of the reagents. Accordingly, one aspect of the present invention relates to kits useful to conveniently performing the assay methods of the invention for the determination of the cholinesterase activity. Within the scope of the present is a kit for determining the activity of a cholinesterase in a sample comprising in a packaged combination a first reagent comprising a buffer solution suitable for the activity of cholinesterase, and a second reagent comprising a stabilized cholinesterase substrate solution, wherein at least one substrate is stabilized by at least one species of a polar organic solvent Also within the scope of the present invention is a kit for conducting a method for determining the activity of a cholinesterase in a sample, the method comprising the steps of combining a sample suspected of containing the enzyme under conditions suitable for enzymatic activity and a reagent comprising a stabilized cholinesterase substrate solution, wherein at least one substrate is stabilized by at least one species of a polar organic solvent, and of monitoring the activity of the enzyme. In an embodiment of the present invention, said kit for conducting said method for determining the activity of a cholinesterase in a sample comprises in a packaged combination a first reagent comprising a buffer solution suitable for the activity of cholinesterase, and a second reagent comprising a stabilized cholinesterase substrate solution, wherein at least one substrate is stabilized by at least one species of a polar organic solvent.

The reagents may remain in liquid form or lyophilized. The kit can further comprise other packaged calibration materials, such as a calibration reagent comprising a known amount of cholinesterase. Calibration material means any standard or reference material containing a known amount of the analyte to be measured. The sample suspected of containing the analyte and the calibration material are assayed under similar conditions. Analyte concentration is then calculated by comparing the results obtained for the unknown specimen with results obtained for the standard. In a preferred embodiment, the kit comprises a buffer solution optimized for cholinesterase activity and a cholinesterase substrate solution stabilized by at least 0.25 volume percent and maximal 100 volume percent of DMSO, at least 0.25 volume percent and maximal 100 volume percent of ethanol, at least 0.5 volume percent and maximal 95 volume percent of isopropanol and/or at least 0.5 volume percent and maximal 100 volume percent of THF. In another preferred embodiment of the present invention, the kit comprises a buffer solution optimized for cholinesterase activity and a cholinesterase substrate solution stabilized by at least 0.25 volume percent and maximal 20 volume percent of DMSO, at least 0.25 volume percent and maximal 20 volume percent of ethanol, at least 0.5 volume percent and maximal 20 volume percent of isopropanol and/or at least 0.5 volume percent and maximal 20 volume percent of THF.

Various ancillary material will frequently be employed in a substrate solution or in an assay in accordance with the present invention. For example, buffers will normally be present in the assay medium, as well as stabilizers for the assay medium and the assay components. Frequently, in addition to these additives, additional proteins may be included, such as albumin, or surfactants, particularly non-ionic surfactants, or the like.

The present invention is now described in more detail with the following examples or figures to which it is of course not limited.

## Examples

### Example 1:

### Determination of cholinesterase activity

Determination of cholinesterase (CHE) activity was based on the formation and detection ofthiocholine according to the following reaction scheme:

butyrylthiocholine + $H_2O$ $\xrightarrow{\text{Cholinesterase}}$ butyric acid + thiocholine

2 thiocholine + + 2 $OH^-$ + $[Fe(CN)6]^{3-}$ $\longrightarrow$ dithiobis(choline) + H2O + 2[ $Fe(CN)6]^{4-}$

Dependent on the cholinesterase activity, thiocholine is specifically liberated from butyrylthiocholine. Thiocholine instantaneously reduces yellow hexacyanoferrate III to almost colourless hexacyanoferrate II. The decrease of extinction can be spectrophotometrically measured at a wavelength of 405 nm and a temperature of 37°C. The cholinesterase concentration is calculated with the molar absorption coeffizient of potassium hexacyanoferrate III (92.7 +/- 0.4 $m^2$/mol).

In particular the determination of cholinesterase activity was performed as follows:

Two reagent solutions, R1 and R2 were freshly prepared:

R1: 91.5 mM Pyrophosphate
2.44 mM Potassium hexacyanoferrate
pH adjusted to 7.7 with citric acid

R2: 10 mM HEPPS
45.7 mM Butyrylthiocholine iodide
pH adjusted to 4.0 with phosphoric acid
polar organic solvent, e.g. 10% (Vol.) DMSO

Serum, heparin or EDTA plasma as sample material or 0.9% (w/v) NaCl, R1 and R2 were pipetted into a cuvette according to the scheme below, resulting in a final concentration of 75.2 mM pyrophosphate, 2,0 mM hexacyanoferrate (III), 7.5 mM butyrylthiocholine and 1.6 mM HEPPS.

Table 1: Pipetting scheme for Reagent Blank and Sample.

| | Reagent Blank | Sample |
|---|---|---|
| Reagent 1 | 900 µl | 900 µl |
| 0.9% NaCl | 15µl | --- |
| Sample | --- | 15µl |

All reagents and solutions mentioned were used as 37°C tempered working solutions. Accordingly the temperature of the mixture in the cuvette again was controlled to assure a temperature of 37 ± 0.05 °C after pipetting.
Then 180 µl R2 was added and the reaction was started by thoroughly mixing the sample.
Using a manual photometer, the spectrophotometrical measurement was performed exactly 90, 120, 150 and 180 sec after addition of R2 according to the following conditions:

| | |
|---|---|
| Temperature: | 37±0.05°C |
| Wavelength: | 405 nm |
| Bandwidth: | 2 nm |
| Light pathway: | 10 mm |
| Delay time: | 90 sec |
| Measuring time | 90 sec |

Further, the following dilution limit for the sample material was taken into account:

$$\Delta E/min = 0.380 \text{ (measuring time: 60 sec)}$$

In case of higher activities 100 µl of the sample was mixed with 100 µl 0,9 %
(w/v) NaCl-solution, the measurement was repeated and the result was multiplied with factor 2.
In case of activities of ΔE/min < 0.007, the sample volume was doubled (30µl), the reagent blank was substracted and the result multiplied with factor 33914.

The measurement was performed against air in order to determine a decrease of extinction. For each series of measurements values for blank reactions were determined 5 times and at any one time values for blank reactions were substracted from the value of the actual enzymatic reaction.

Thus the turnover rate of cholinesterase in the sample equals the difference of the sample reaction and the blank reaction:

$$(\Delta E/min)CHE = (\Delta E/min) \text{ Sample} - (\Delta E/min) \text{ Reagent Blank}$$

The catalytical concentration of cholinesterase (U/L) was calculated by multiplying the turnover rate (ΔE/min) CHE with the factor 78749:

$$U/L = 78749 \cdot \Delta E_{CHE}/min$$

whereby: 1000 U/L = 16.67 µkat/L; 1 µkat/L = 60 U/L,

The formula with the factor 78749 is based on the following calculation:

At the wavelength of 405 nm and a measuring temperature of 37°C the catalytical concentration is:

$$b = A \cdot \frac{V}{\varepsilon \cdot l \cdot v}$$

- molar absorption coeffizient of Potassium hexacyanoferrate at 405nm:

$$\varepsilon = 92{,}7\ m^2 \cdot mol^{-1} \text{ (see DGKC proposal)}$$

- light pathlength, *l*: 0,01 m
- reaction volume, *V:* $1{,}095 \cdot 10^{-3}$ L
  (R1: $0.900 \cdot 10^{-3}$ L + R2: $0.180\text{-}10^{-3}$ L + sample)
- Sample volume, ν:$0{,}015 \cdot 10^{-3}$ L
- A: decrease of extinction for 60 sec fit in:

$$b = A \cdot \frac{1{,}095 \cdot 10^{-3}}{92{,}7 \cdot 0{,}01 \cdot 0{,}015 \cdot 10^{-3}} \cdot \frac{L \cdot mol}{\cdot m^2 \cdot m \cdot L \cdot \min}$$

$$b = A \cdot 78{,}7487 \cdot \frac{mol}{m^3 \cdot min}$$

$$b = A \cdot 78749 \cdot \frac{U}{L} \quad \text{or} \quad b = A \cdot 1312{,}5 \cdot \frac{\mu kat}{L} \quad (1\mu kat = 60U)$$

The experiments in the following examples were performed according to an analogous procedure using an automated Roche/Hitachi 917 analyzer with the pipetting volumes: R1: 180μl, Sample: 3μl, and R2: 36μl at a wavelength of 415nm.

**Example 2:**

**Assessment of the stability of butyrylthiocholine (STC) with different polar organic solvents**

Insufficient stabilisation of BTC could be seen in a change of the results by aging of the reagent. For the assessment of the BTC stability the catalytical concentration of cholinesterase obtained with tempered substrate solution (tempered at 35°C for 18-21 days) was compared with that obtained with freshly prepared substrate solution. Measurements were conducted on a Roche/Hitachi 917 analyzer analogous to the procedure described in example 1.

The above-mentioned "aging model" is appropriate to evaluate quickly different cholinesterase substrate solutions since a reagent tempered at 35°C for 18-21 days corresponds to a reagent stored under refrigerated routine conditions for 15-18 months.

Using this "aging model" the stability of different cholinesterase substrate solutions - /+ certain polar organic solvents was determined according to the intercept and the slope of a straight-line in a plot of CHE catalytic concentrations obtained using freshly prepared and tempered substrate solution (see e.g. Figs. 1, 2, and 3).

These parameters (intercept and slope) were compared with the parameters of a not stabilized cholinesterase substrate solution, e.g. prepared as proposed by the DGKC (Deutsche Gesellschaft für Klinische Chemie, see Schmidt et al., Eur. J. Clin. Chem. Clin. Biochem. 30(3): 163-170 (1992) (see Fig. 2) or prepared according to Example 1 (i.e. R2 without polar organic solvent) (see Fig. 3).

Accordingly an intercept of about < +/- 300 U/l and a slope < +/- 3 % were the criteria for a stabilized cholinesterase substrate solution indicating that there was virtually no loss of BTC during the incubation at 35°C for 21 days.

In contrast, the values of these parameters analogously determined with the not stabilized original formulation as proposed by the DGKC or prepared according to Example 1 (i.e. R2 without polar organic solvent) differ significantly from those indicating stabilization of BTC:

DGKC formulation: y = -1464 + 0.8105*x (see Fig.2)

The fresh reagent was calibrated with the Roche calibrator CFAS and the tempered reagent was read off the calibration curve of the fresh reagent.

Cholinesterase substrate solutions stabilized with the following polar organic solvents were tested and fulfil the requirements for the stabilisation of BTC (each the concentration in volume percent is indicated in brackets):

| Polar organic solvent (vol. %) | straight line function |
|---|---|
| Ethanol (20%) | y = -178 + 1.016*x |
| DMSO (20%) | y = 14 + 0.985*x (see Fig. 1) |
| Isopropanol (20%) | y = 190 + 0.976*x |
| THF (5%) | y = 252 + 0.982*x |
| Acetonitril (20%) | y = 187 + 0.995*x |
| Sulfanol (20%) | y = 58 + 1.020*x |
| Glycerin (20%) | y = 302 + 0.976*x |

**Example 3:**

**Determination of limit concentrations of different polar organic solvents for stability of butyrylthiocholine (BTC)**

Based on the "aging model" as described in Example 2 the minimal concentration (in volume percent) of a polar organic solvent was determined according to the intercept and the slope of a straight-line in a plot of CHE catalytic

concentrations obtained using freshly prepared and tempered substrate solution.

Table 2: Determination of limit concentrations of different polar organic solvents for stability of butyrylthiocholine (BTC).

| | Ethanol | DMSO | Isopropanol | THF |
|---|---|---|---|---|
| 1% | y=29 + 1.000*x | y=-99 + 0.977*x | y=404 + 0.935*x | y=306 + 0.963*x |
| 2% | - | y=80 + 0.977*x | y=316 + 0.967*x | y=256 + 0.974*x |
| 3% | y = 64 + 1.011*x | y=19 + 0.987*x | y=275 + 0.980*x | y=218 + 1.000*x |
| 4% | - | y=-28 + 0.995*x | y=260 + 0,990*x | y=208 + 1.005*x |
| 5% | - | y=68 + 1.006*x | y=277 + 0.985*x | y=252 + 0.982*x |
| 10% | y=301 + 0.974*x | y= -81 + 1.013*x | y=243 + 1.006*x | y=273 + 1.029*x |
| 20% | y=-178 + 1.016*x | y= 14 + 0.985*x | y=180 + 0.976*x | y=267 + 1.069*x |

Taking into account the criteria for a stabilized cholinesterase substrate solution, i.e. an intercept of about < +/- 300 U/l and a slope < +/- 3 %, the lowest concentration with a stabilising effect was 1% (volume percent) for each ethanol and DMSO, and 2% (volume percent) for each THF and Isopropanol.

**Example 4:**

**Assessment of the stability of butyrylthiocholine (BTC) with combinations of different polar organic solvents**

From the data summarized in Table 2 it can be concluded that a stabilized solution of BTC can be prepared using 0.25% DMSO, 0.25% Ethanol, 0.5% Isopropanol and 0.5% THF.

Reagent R1 is prepared according to Example 1. Reagent R2 is prepared according to Example 1 including the polar organic solvents DMSO, 0.25%; ETOH, 0.25%; Isopropanol, 0.5%; THF, 0.5%.

An aliquot of the freshly prepared R2 is stored at 35°C for 21 days. Another aliquot is stored refrigerated at 4-8°C. After this aging period, both aliquots are used together with R1 in the determination of cholinesterase activity according to Example 1 for at least 30 samples with CHE activities between 3000 and 15000 U/1.

The plot of results with the aged R2 versus the R2 stored refrigerated is expected to give results indicating that there was virtually no loss of BTC according to the criteria for a stabilized cholinesterase substrate solution as defined above.

## Claims

1. A stabilized cholinesterase substrate solution, wherein at least one substrate is stabilized by at least one species of a polar organic solvent.

2. The solution of claim 1, wherein the said substrate is a choline ester.

3. The solution of claim 1, wherein the said substrate is a thioester.

4. The solution of claim 3, wherein said thioester is butyrylthiocholine (BTC).

5. The solution of claim 3, wherein said thioester is acetylthiocholine (ATC).

6. The solution of claim 1, wherein said stabilized cholinesterase substrate solution is a stabilized acylcholinesterase substrate solution.

7. The solution of claim 1, wherein said stabilized cholinesterase substrate solution is a stabilized acetylcholinesterase substrate solution.

8. The solution of claim 1, wherein said at least one species of a polar organic solvent is a polar protic organic solvent.

9. The solution of claim 8, wherein said polar protic organic solvent is an alcohol.

**10.** The solution of claim 9, wherein said alcohol is selected from the group with a chain length between 1 and 7 carbon atoms.

**11.** The solution of claim 10, wherein said alcohol is selected from the group consisting of methanol, ethanol, propanol and isopropanol.

**12.** The solution of claim 10, wherein said alcohol is glycerine.

**13.** The solution of claim 1, wherein said at least one species of a polar organic solvent is a polar aprotic organic solvent.

**14.** The solution of claim 13, wherein said polar aprotic organic solvent is selected from the group consisting of carbonyl compounds or heterocarbonyl compounds.

**15.** The solution of claim 14, wherein said carbonyl compound or heterocarbonyl compound is selected from the group containing 1 to 12 carbon atoms.

**16.** The solution of claim 15, wherein said carbonyl compound or heterocarbonyl compound is selected from the group consisting of dimethylsulfoxide (DMSO), dimethylformamid (DMF), acetone, acetylacetone, acetonitril and hexamethylphosphoric triamide (HMPT).

**17.** The solution of claim 13, wherein said polar aprotic organic solvent is an ether.

**18.** The solution of claim 17, wherein said ether is selected from the group containing 1 to 12 carbon atoms.

**19.** The solution of claim 18, wherein said ether is a cyclic ether.

**20.** The solution of claim 19, wherein said cyclic ether is selected from the group consisting of tetrahydrofuran (THF) and dioxan.

**21.** The solution of claim 1, wherein the concentration of said at least one species of a polar organic solvent in said reaction mixture is at least 0.25 volume percent and maximal 100 volume percent.

**22.** The solution of claim 1, wherein the concentration of said at least one species is for DMSO at least 0.25 volume percent and maximal 100 volume percent, for ethanol at least 0.25 volume percent and maximal 100 volume percent, for isopropanol at least 0.5 volume percent and maximal 95 volume percent, and/or for THF at least 0.5 volume percent and maximal 100 volume percent.

**23.** The solution of claim 1, wherein the concentration of said at least one species is for DMSO at least 0.25 volume percent and maximal 20 volume percent, for ethanol at least 0.25 volume percent and maximal 20 volume percent, for isopropanol at least 0.5 volume percent and maximal 20 volume percent, and/or for THF at least 0.5 volume percent and maximal 20 volume percent.

**24.** The use of a stabilized cholinesterase substrate solution, wherein at least one substrate is stabilized by at least one species of a polar organic solvent.

**25.** A stabilized cholinesterase substrate solution for determining the activity of a cholinesterase in a sample, wherein at least one substrate is stabilized by at least one species of a polar organic solvent.

**26.** The solution of claim 25, wherein said sample is a biological sample.

**27.** The solution of claim 25, wherein said cholinesterase is acylcholinesterase.

**28.** The solution of claim 25, wherein said cholinesterase is acetylcholinesterase.

**29.** A kit for determining the activity of a cholinesterase in a sample, comprising in a packaged combination:

a. a first reagent comprising a buffer solution suitable for the activity of said enzyme,
b. a second reagent according to claim 1.

**30.** The kit of claim 29, said kit further comprising a calibration reagent comprising a known amount of said enzyme.

**31.** The kit of claim 29, wherein said sample is a biological sample.

**32.** The kit of claim 29, wherein said cholinesterase is acylcholinesterase.

**33.** The kit of claim 29, wherein said cholinesterase is acetylcholinesterase.

**34.** A method for determining the activity of a cholinesterase in a sample comprising the steps of:

a. combining a sample suspected of containing the enzyme under conditions suitable for enzymatic activity and a reagent according to claim 1,
b. monitoring the activity of the enzyme.

**35.** The method of claim 34, wherein said sample is a biological sample.

**36.** The method of claim 34, wherein said cholinesterase is acylcholinesterase.

**37.** The method of claim 34, wherein said cholinesterase is acetylcholinesterase.

**38.** The method of claim 34, wherein said monitoring of the activity of the enzyme is conducted by determining the formation of thiocholine.

**39.** The method of claim 38, wherein said formation of thiocholine is determined by measuring the reduction of hexacyanoferrate (III) to hexacyanoferrate (II) by spectrophotometry.

**40.** The method of claim 34, wherein said monitoring of the activity of the enzyme is conducted using an automated analyzer.

**41.** A kit for conducting the method of claim 34 comprising in a packaged combination:

a. a first reagent comprising a buffer solution suitable for enzymatic activity, and
b. a second reagent according to claim 1.

**42.** The kit of claim 41, said kit further comprising a calibration reagent comprising a known amount of said enzyme.

**Amended claims in accordance with Rule 86(2) EPC.**

**1.** Use of a polar organic solvent for the stabilization of a cholinesterase substrate solution, wherein at least one substrate is stabilized by at least one species of a polar organic solvent in a buffered solution.

**2.** The use of claim 1, wherein the said substrate is a choline ester.

**3.** The use of claim 1, wherein the said substrate is a thioester.

**4.** The use of claim 3, wherein said thioester is butyrylthiocholine (BTC) or acetylthiocholine (ATC).

**5.** The use of claim 1, wherein said cholinesterase substrate solution is an acylcholinesterase substrate solution or an acetylcholinesterase substrate solution.

**6.** The use of claim 1, wherein said at least one species of a polar organic solvent is a polar protic organic solvent.

**7.** The use of claim 6, wherein said polar protic organic solvent is an alcohol.

**8.** The use of claim 7, wherein said alcohol is selected from the group with a chain length between 1 and 7 carbon atoms.

**9.** The use of claim 8, wherein said alcohol is selected from the group consisting of methanol, ethanol, propanol, isopropanol and glycerine.

**10.** The use of claim 1, wherein said at least one species of a polar organic solvent is a polar aprotic organic solvent.

**11.** The use of claim 10, wherein said polar aprotic organic solvent is selected from the group consisting of carbonyl compounds and heterocarbonyl compounds.

**12.** The use of claim 11, wherein said carbonyl compound or heterocarbonyl compound is selected from the group containing 1 to 12 carbon atoms.

**13.** The use of claim 12, wherein said carbonyl compound or heterocarbonyl compound is selected from the group consisting of dimethylsulfoxide (DMSO), dimethylformamid (DMF), acetone, acetylacetone, acetonitril and hexamethylphosphoric triamide (HMPT).

**14.** The use of claim 10, wherein said polar aprotic organic solvent is an ether.

**15.** The use of claim 14, wherein said ether is selected from the group containing 1 to 12 carbon atoms.

**16.** The use of claim 15, wherein said ether is a cyclic ether.

**17.** The use of claim 16, wherein said cyclic ether is selected from the group consisting of tetrahydrofuran (THF) and dioxan.

**18.** The use of claim 1, wherein the concentration of said at least one species of a polar organic solvent is for DMSO at least 0.25 volume percent and maximal 20 volume percent, for ethanol at least 0.25 volume percent and maximal 20 volume percent, for isopropanol at least 0.5 volume percent and maximal 20 volume percent, and/or for THF at least 0.5 volume percent and maximal 20 volume percent.

**19.** The use of one of the previous claims, wherein said substrate is not significantly inactivated after a storage at 35°C over a time period of at least 18 days.

**20.** The use of claim 1 to 18, wherein said substrate is not significantly inactivated after a storage at 4-8°C for a time period of at least 15 months.

**21.** A kit for determining the activity of a cholinesterase in a sample comprising in a packaged combination:

a. a first reagent comprising a buffer solution suitable for the activity of said enzyme,
b. a second reagent comprising a stabilized cholinesterase substrate solution, wherein at least one substrate is stabilized by at least one species of a polar organic solvent in a buffered solution.

**22.** The kit of claim 21, said kit further comprising a calibration reagent comprising a known amount of said enzyme.

**23.** The kit of claim 21, wherein said sample is whole blood, plasma or serum.

**24.** The kit of claim 21, wherein said cholinesterase is acylcholinesterase or acetylcholinesterase.

**25.** The kit of claim 21, wherein said substrate is not significantly inactivated after a storage at 35°C over a time period of at least 18 days.

**26.** The kit of claim 21, wherein said substrate is not significantly inactivated after a storage at 4-8°C for a time period of at least 15 months.

**Figure 1**

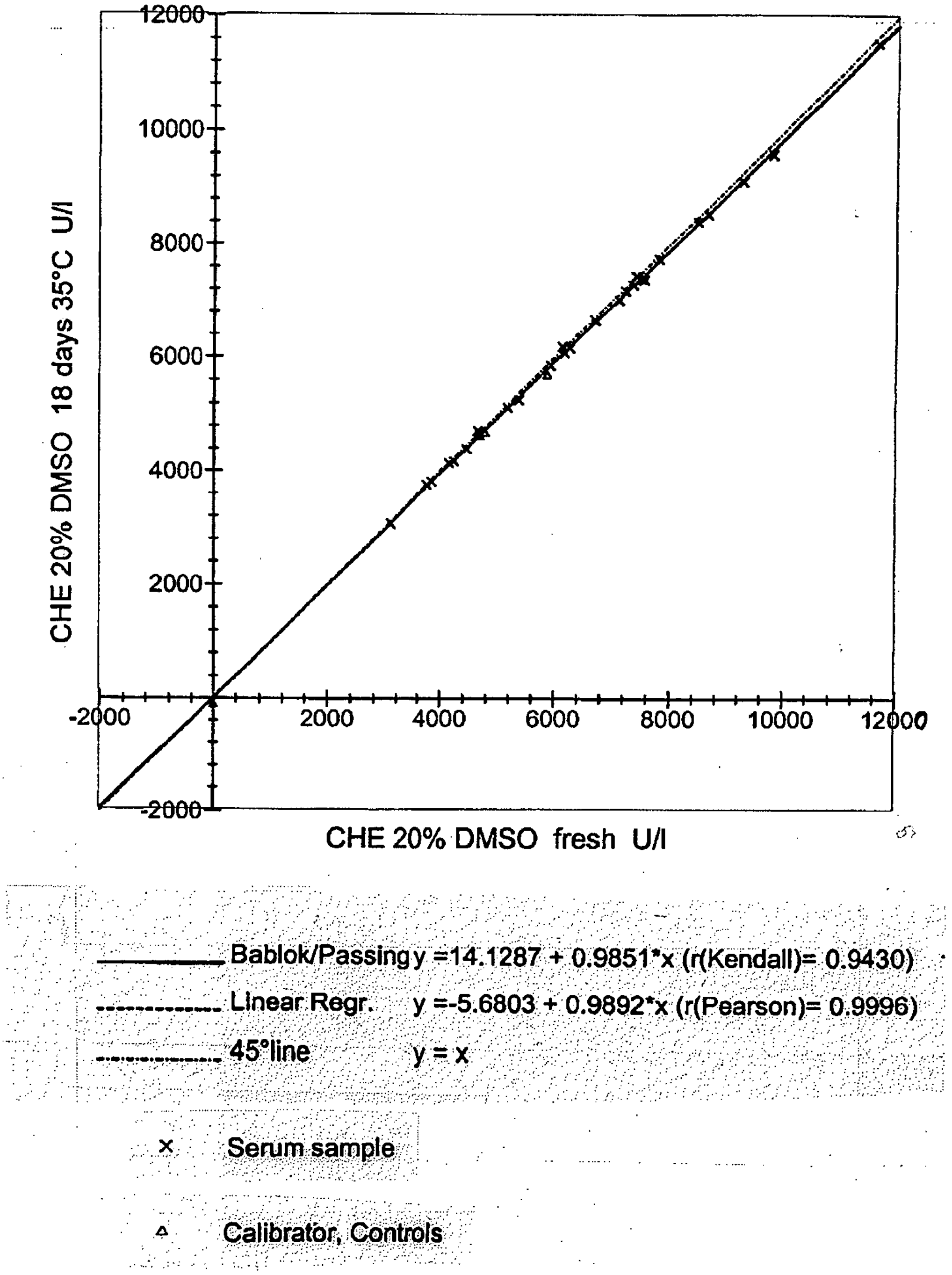
12000
10000
8000
6000
4000
2000
-2000
-2000
2000
4000
6000
8000
10000
12000
CHE 20% DMSO 18 days 35°C U/l
CHE 20% DMSO fresh U/l
Bablok/Passing y =14.1287 + 0.9851*x (r(Kendall)= 0.9430)
Linear Regr. y =-5.6803 + 0.9892*x (r(Pearson)= 0.9996)
45°line y = x
Serum sample
Calibrator, Controls

**Figure 2**

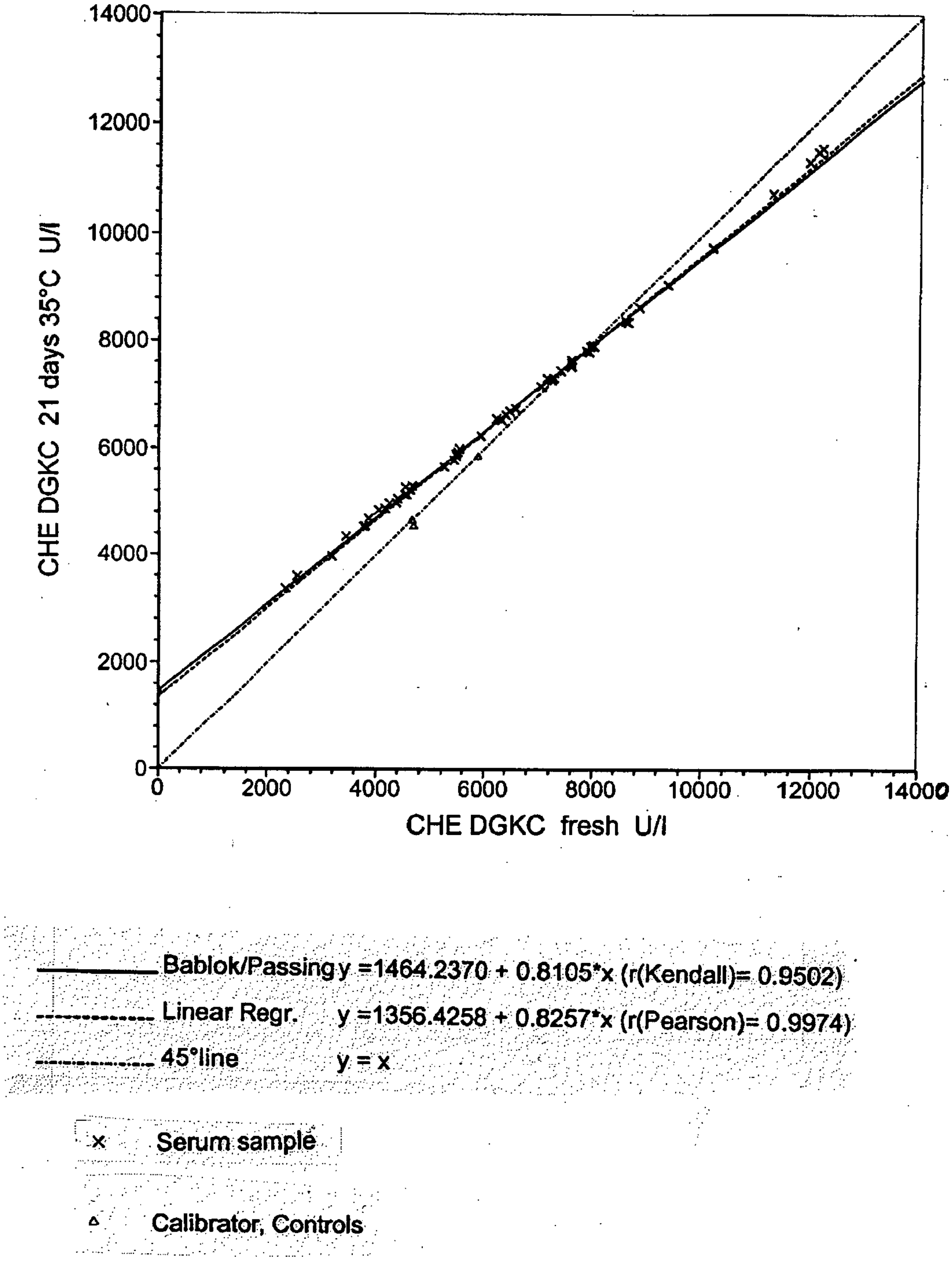
CHE DGKC 21 days 35°C U/l
0
2000
4000
6000
8000
10000
12000
14000
CHE DGKC fresh U/l
Bablok/Passing y =1464.2370 + 0.8105*x (r(Kendall)= 0.9502)
Linear Regr. y =1356.4258 + 0.8257*x (r(Pearson)= 0.9974)
45°line y = x
Serum sample
Calibrator, Controls

European Patent Office

# EUROPEAN SEARCH REPORT

Application Number
EP 05 02 6822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 401 086 A (HOFFMANN ARNO ET AL) 10 September 1968 (1968-09-10) * the whole document * | 1-42 | C12Q1/46 |
| X | US 3 433 712 A (HORACE W. GERARDE) 18 March 1969 (1969-03-18) * the whole document * | 1-42 | |
| X | US 2004/101923 A1 (LEE HAN LIM ET AL) 27 May 2004 (2004-05-27) * the whole document * | 1-42 | |
| X | US 2004/137550 A1 (LEE HAN LIM ET AL) 15 July 2004 (2004-07-15) * the whole document * | 1-42 | |
| A | OSAWA S ET AL: "Development and application of serum cholinesterase activity measurement using benzoylthiocholine iodide" CLINICA CHIMICA ACTA, AMSTERDAM, NL, vol. 351, no. 1-2, January 2005 (2005-01), pages 65-72, XP004654505 ISSN: 0009-8981 | | TECHNICAL FIELDS SEARCHED (IPC) |
| A | SCHMIDT E ET AL: "Proposal of standard methods for the determination of enzyme catalytic concentrations in serum and plasma at 37[deg.]C. II. Cholinesterase (acylcholine acylhydrolase, EC 3.1.1.8)" EUROPEAN JOURNAL OF CLINICAL CHEMISTRY AND CLINICAL BIOCHEMISTRY 1992 GERMANY, vol. 30, no. 3, 1992, pages 163-170, XP009060980 ISSN: 0939-4974 | | C12Q |

The present search report has been drawn up for all claims

| Place of search | Date of completion of the search | Examiner |
|---|---|---|
| Munich | 6 February 2006 | Luo, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 05 02 6822

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2006

| Patent document cited in search report | Publication date | Patent family member(s) | Publication date |
|---|---|---|---|
| US 3401086 A | 10-09-1968 | BE 675489 A | 25-07-1966 |
| | | CH 447661 A | 30-11-1967 |
| | | DE 1598739 A1 | 07-01-1971 |
| | | GB 1060899 A | 08-03-1967 |
| | | NL 6600994 A | 27-07-1966 |
| US 3433712 A | 18-03-1969 | FR 1462521 A | 01-03-1967 |
| | | GB 1116539 A | 06-06-1968 |
| | | GB 1116540 A | 06-06-1968 |
| US 2004101923 A1 | 27-05-2004 | CN 1502982 A | 09-06-2004 |
| | | EP 1439389 A2 | 21-07-2004 |
| | | JP 2004173654 A | 24-06-2004 |
| US 2004137550 A1 | 15-07-2004 | CN 1508262 A | 30-06-2004 |
| | | GB 2397125 A | 14-07-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BROWN et al.** *Adv. Clin. Chem.,* 1981, vol. 22, 1-123
- Tietz fundamentals of clinical chemistry
- **WHITTAKER M.** Methods of Enzymatic Analyses. Weinheim Verlag Chemie, 1984, 52-74
- **DIETZ et al.** Colorimetric Determination of Serum Cholinesterase and Its Genetic Variance by the Propionylthiocholine-Dithiobis(nitrobenzvic Acid) Procedure. *Clinical Chemistry,* 1973, vol. 19, 1309-1313
- **SCHMIDT E. et al.** Proposal of Standard Methods for the Determination of Enzyme Catalytic Concentrations in Serum and Plasma at 37°C. *Eur.J.Clin.Chem.Clin.Biochem.,* 1992, vol. 30, 163-170
- **KNEDEL ; BÖTTGER.** *Klin Wschr,* 1967, vol. 45, 325-327
- News Sheet Assoc. Clin. Biochemists. 1980, 31s-36s
- Enzym-Aktivitätsbestimmung in der Laboratoriumsdiagnostik. Akademie-Verlag, 1988, 56-65
- **KNEDEL ; BÖTTGER.** *Klin. Wschr.,* 1967, vol. 45, 325-327
- **SCHMIDT et al.** *Eur. J. din. Chem. Clin. Biochem.,* 1992, vol. 30 (3), 163-170
- **SCHMIDT et al.** *Eur. J. Clin. Chem. Clin. Biochem.,* 1992, vol. 30 (3), 163-170